Europäisches Patentamt

European Patent Office

Office européen des brevets

(19)

(11) Publication number: **0 450 717 A2**

# EUROPEAN PATENT APPLICATION

(12)

(21) Application number: **91200730.9**

(22) Date of filing: **28.03.91**

(51) Int. Cl.5: **C07F 9/00**

(30) Priority: **05.04.90 US 505316**

(43) Date of publication of application:
**09.10.91 Bulletin 91/41**

(84) Designated Contracting States:
**DE FR GB NL**

(71) Applicant: **AKZO N.V.**
**Velperweg 76**
**NL-6824 BM Arnhem(NL)**

(72) Inventor: **Greco, Carl Cataldo**
**53 Main Street**
**Garnerville, New York 10923(US)**

(74) Representative: **Schalkwijk, Pieter Cornelis et al**
**Akzo Patents Department P.O. Box 314**
**NL-6800 AH Arnhem(NL)**

(54) Niobium alkoxyalkoxides and process for formation.

(57) Hydrocarbon soluble niobium alkoxy alkoxides can be formed by the reaction of a niobium dialkylamide with an alkoxy alcohol is a hydrocarbon solvent medium.

EP 0 450 717 A2

## Background of the invention

U.S. Patent No. 3,277,002 to M.W. Hunt et al. describes metal alkoxide formation by reaction of a sodium alkoxide with a metal compound. It indicates that Group VB metals can be selected and names vanadium. The alcohol used to form the sodium alkoxide can be an alkoxy alkanol such as an alkoxy ethanol. The Hunt disclosure does not name niobium as a metal and does not exemplify the synthesis of niobium alkoxides of any type.

U.S. Patent No. 3,558,521 to H. E. Berkheimer et al. illustrates the preparation of tetrakis-(beta-isobutoxyethoxy) vanadium from tetrakis-(diethylamino) vanadium. D. C. Bradley et al. in Can. J. Chem., Vol. 40, pp. 1183-1188 (1962) indicate that tetrakis (diethylamino) vanadium (IV) can be used in the synthesis of vanadium tetraalkoxides but that such compounds are rather unstable. Niobium alkoxides are not alluded to in either of these references as well. I. M. Thomas, in Can. J. Chem., Vol. 39, 1386-1388 (1961) indicates that the dialkylamides of titanium, zirconium, vanadium, niobium, tantalum and tin are very reactive with compounds containing replaceable hydrogen atoms. This method is said to be applicable to the preparation of all alkoxides of vanadium (IV) and tin and to the "tertiary" alkoxides of zirconium and niobium (i.e., niobium penta(isopropoxide)). This reference also distinguishes between the behavior of vanadium and niobium metal in alkoxide formation by indicating that it was not possible to form the tetraalkoxide of niobium but that it was successful with vanadium.

More recent publications which discuss more complicated niobium alkoxides or niobium alkoxide complexes include: Inorg. Chem. 1987, 26, 3319-3323 (which discusses dinuclear niobium (IV) complexes comprising chlorine and methoxy); Inorg. Chem. 1987, 26, 3323-3327 (which discusses dimeric niobium (III) alkoxide compounds also comprising chlorine and alkyl-containing alkoxide ligands); and Inorg. Chem. 1988, 27, 3596-3600 (which discusses chlorine-containing low valent complexes of niobium and dimeric niobium (IV) nonamethoxide).

## Summary of the invention

Thepresentinventionrelates tonobiumalkoxyalkoxideswhich are soluble in hydrocarbon solvent as well as to a process for their synthesis which comprises reaction of the chosen alkoxy alkanol with a niobium dialkylamide.

## Description of the invention

The first step in regard to the present invention is the formation of a suitable niobium dialkylamide reagent which can be reacted with an alkoxy alcohol to form the desired hydrocarbon soluble niobium alkoxyalkoxides.

The niobium dialkylamide is of the formula

$$Nb(NR_2)_4$$

where R can be lower alkyl of from 1 to 4 carbon atoms such as methyl or ethyl. This reagent is advantageously formed by reacting an alkali metal dialkylamide (e.g., a lithium dialkylamide such as lithium diethylamide) with a niobium compound (e.g., such as a niobium tetrachloride) in a suitable hydrocarbon solvent. Example 1 illustrates a representative procedure which results in the formation of a niobium dialkylamide product which can be dissolved in a suitable organic solvent (e.g., toluene) for reaction with an alkoxy alcohol.

The niobium dialkyl amide prepared as described above is then reacted with a suitable alkoxy alcohol of the general formula

$$R''OR'OH$$

where R' is alkylene of from about 2 to about 3 carbon atoms (e.g., propylene) and R" is alkyl of from 1 to about 8 carbon atoms (e.g., methyl) to form the desired hydrocarbon soluble niobium alkoxides of the formula

$$Nb(OR'OR'')_4$$

where R' and R" are as defined above.

The hydrocarbon solutions described herein can be used to coat a desired substrate (e.g., the nose cones of space vehicles) to deposit a coating thereon which, when pyrolyzed, forms a protective niobium oxide coating on the substrate.

The present invention is further illustrated by the Examples which follow.

## EXAMPLE 1

Into a 500 ml, three neck flask was added 22 gm of lithium diethylamide (0.28 mole) and 250 cc of dry tetrahydrofuran. To this was added 19.7 gm of niobium tetrachloride-tetrahydrofuran complex dissolved in 150 cc of tetrahydrofuran. The amount of niobium tetrachloride used was 0.052 mole and was added dropwise over a fifteen minute period. The reaction mixture was exothermic and rose 15 o C over the course of the addition. After the addition had been completed, the reaction mixture was refluxed for three hours. The reaction mixture was then distilled to dryness. A dark brown-black oil remained as the product (14.2 gm; theoretical amount 19.8 gm). The oil was dissolved in 400 cc of toluene and used in the next Example. Chloride analysis of the solution showed less than 0.04%, by weight.

EXAMPLE 2

The niobium solution from Example 1 containing 0.86%, by weight, niobium was reacted with 40.4 gm of methoxypropanol. The alkoxyalcohol was added over a fifteen minute period where the reaction temperature rose 10 o C. The solution was allowed to stir overnight at room temperature. The next day vacuum (20 mm of Hg) was applied to remove the diethylamine by-product. The solution was collected as the product. Analysis showed 1.8%, by weight, niobium (66% of theory).

The foregoing Examples are intended to be illustrative of only certain embodiments of the present invention and should not therefore be construed in a limiting sense. The scope of protection sought is set forth in the claims which follow.

## Claims

1. Hydrocarbon soluble niobium alkoxy alkoxides.

2. Alkoxy alkoxides as claimed in Claim 1 having the formula $Nb(OR'OR'')_4$ where R' is alkylene and R'' is alkyl.

3. Alkoxy alkoxides of the formula of Claim 2 wherein R' is C 2 to C 3 alkylene and R'' is C 1 to C 8 alkyl.

4. Niobium methoxypropoxide.

5. A process for the synthesis of the alkoxy alkoxides of Claim 1 which comprises reacting an alkoxy alcohol with a niobium dialkylamide.

6. A process as claimed in Claim 5 wherein the alkoxy alcohol is of the formula R''OR'OH where R' is alkylene and R'' is alkyl.

7. A process as claimed in Claim 5 wherein the reaction is carried out in hydrocarbon solvent.

8. A process as claimed in Claim 6 wherein R' is C 2 to C 3 alkylene and R'' is C 1 to C 8 alkyl.

9. A process as claimed in Claim 5 wherein the alkoxy alcohol is methoxy propanol.

10. Hydrocarbon solutions containing an alkoxy alkoxide of niobium as claimed in any one of Claims 1-4 dissolved therein.